# EUROPEAN PATENT APPLICATION

(11) **EP 2 551 279 A1**
(43) Date of publication of application: **30.01.2013**
(21) Application number: 11175589.8
(22) Date of filing: 27.07.2011
(51) Int. Cl.: C07K 14/335, A61K 35/74, A23L 1/30

(54) **Antimicrobial peptide produced by intestinal Lactobacillus salivarius**

(71) Applicant: University College Cork, Cork City (IE); Agriculture and Food Development Authority (TEAGASC), Carlow (IE)
(72) Inventor: Ross, R. Paul, Co. Cork (IE); O'Shea, Eileen F., Co. Cork, Banteer (IE); Hill, Colin, Cork (IE)
(74) Representative: Purdy, Hugh Barry

(57) **Abstract**

An antimicrobial peptide having potent antibacterial activity against *Listeria monocytogenes* and *Staphylococcus aureus* is described. Also described is an isolated *Lactobacillus salivarius* DPC6502 strain as deposited with the National Collection of Industrial and Marine Bacteria under the Accession No. NCIMB 41840, and variants thereof, wherein the isolated bacteria and variants thereof express an antimicrobial peptide having potent antibacterial activity against *Lactobacillus delbrueckii* subspecies *bulgaricus, Listeria* and *Staphylococcus aureus.*

SEQUENCE LISTING

<110>  University College Cork - National University of Ireland,
       Cork
       Agriculture and Food Development Authority (Teagasc)

<120>  Antimicrobial peptide produced by intestinal Lactobacillus
       salivarius

<130>  P10552EP00

<160>  13

<170>  PatentIn version 3.5

<210>  1
<211>  69
<212>  DNA
<213>  Lactobacillus salivarius

<400>  1
aagagaaaga aacatcgttg cagagtttat aataatggaa tgcctacagg aatgtatcgt      60

tggtgctaa                                                              69


<210>  2
<211>  22
<212>  PRT
<213>  Lactobacillus salivarius

<400>  2

Lys Arg Lys Lys His Arg Cys Arg Val Tyr Asn Asn Gly Met Pro Thr
1               5                   10                  15


Gly Met Tyr Arg Trp Cys
            20


<210>  3
<211>  29
<212>  DNA
<213>  Artificial Sequence

<220>
<223>  Forward Primer sequence

<400>  3
cagtcgacaa tgatcatgat ggagtagcg                                        29


<210>  4
<211>  29
<212>  DNA
<213>  Artificial Sequence

<220>
<223>  Reverse Primer sequence

<400>  4
ggaagtaagt agggttttga taaggtgtc                                        29


<210>  5

<211>  22
<212>  PRT
<213>  Lactobacillus salivarius

<220>
<221>  misc_feature
<223>  X at positions 4 and 7 indicate blank cycles during Edman
       analysis of the purified peptide for which no amino acid
       derivative was detected.

<220>
<221>  misc_feature
<222>  (4)..(4)
<223>  Xaa can be any naturally occurring amino acid

<220>
<221>  misc_feature
<222>  (7)..(7)
<223>  Xaa can be any naturally occurring amino acid

<400>  5

Lys Arg Lys Xaa His Arg Xaa Arg Val Tyr Asn Asn Gly Met Pro Thr
1               5                   10                  15

Gly Met Tyr Arg Tyr Met
            20

<210>  6
<211>  12
<212>  PRT
<213>  Lactobacillus salivarius

<220>
<221>  MISC_FEATURE
<223>  consensus sequence of double glycine leader sequence

<400>  6

Val Ser Arg Lys Asp Leu Ala Lys Val Asn Gly Gly
1               5                   10

<210>  7
<211>  1191
<212>  DNA
<213>  Lactobacillus salivarius

<400>  7
atgtttagtt tgacacctta tcaaaaccct acttacttcc tattattagg aatatttttt       60

attccaatta tatttggaat tcttaatggt agaagatttc ggtggtatga aacaatagtt      120

tctgtttatt ttctctatat gtcgtttggt ggtactaaat gggagcaagg tgtggcatta      180

atttgttatc tgctttttga ggtgatttta gtaactgcct ataataaata taggaagaaa      240

agaaattctt tccagatatt tttaatggtc actatattat cgattctacc tttgattata      300

gtgaaaataa cgcctttctt aggaatgaaa tcaatttttg gatttttagg aataagttat      360

ttaaccttta aagcagttca aacagttatg gaaataagag atggggttat aaaagacttt 420

aatccatggt tttttttgaa ttttttggct ttttttccaa ccatatcttc aggtccaatt 480

gatcgttata gaaggtataa aaaggattat tatagtgttc ctaataaaga aaaatatatc 540

caattattag aaaagggatt gcactatata tttttaggtt ttttatatga ttttatgttg 600

tcatatttct ttggtacagt gttacttccg ggaataaaga gagaagtaat agcttctact 660

ggagtatcgt tagctttagt agaatacatg tatgtatata gtatgtatct tttctttaat 720

tttgctggat atagtttatt tgcagtagga acaagctatt ttatgggaat tgaaacaccg 780

atgaatttta atcaaccatt taaatctaaa aatataaaag aattttggaa tagatggcac 840

atgacactat ctttttggtt tagagattat gtatatatga gacttgtgtt tttctttatg 900

aagaaaaaag tatttaagaa tcctaaaact acagccaata taacttatat tttaaatatg 960

ttactaatgg gattttggca tggtgaaaca tggtactata tactttatgg ttttatacat 1020

ggcgttgcat tagtagtaaa tgattggtgg ttgggatata aaaagaaaca taaggatgtt 1080

gtaccacata ataaatttac tgagctattt gctatattta taacgttcaa ttttgtttgt 1140

tttacatttt taatttttag tggtatttta gattttgtaa agtttagata a 1191

<210> 8
<211> 396
<212> PRT
<213> Lactobacillus salivarius

<400> 8

Met Phe Ser Leu Thr Pro Tyr Gln Asn Pro Thr Tyr Phe Leu Leu Leu
1               5                   10                  15

Gly Ile Phe Phe Ile Pro Ile Ile Phe Gly Ile Leu Asn Gly Arg Arg
            20                  25                  30

Phe Arg Trp Tyr Glu Thr Ile Val Ser Val Tyr Phe Leu Tyr Met Ser
        35                  40                  45

Phe Gly Gly Thr Lys Trp Glu Gln Gly Val Ala Leu Ile Cys Tyr Leu
    50                  55                  60

Leu Phe Glu Val Ile Leu Val Thr Ala Tyr Asn Lys Tyr Arg Lys Lys
65                  70                  75                  80

Arg Asn Ser Phe Gln Ile Phe Leu Met Val Thr Ile Leu Ser Ile Leu
                85                  90                  95

Pro Leu Ile Ile Val Lys Ile Thr Pro Phe Leu Gly Met Lys Ser Ile
            100                 105                 110

Phe Gly Phe Leu Gly Ile Ser Tyr Leu Thr Phe Lys Ala Val Gln Thr

        115                 120                 125

Val Met Glu Ile Arg Asp Gly Val Ile Lys Asp Phe Asn Pro Trp Phe
    130                 135                 140

Phe Leu Asn Phe Leu Ala Phe Phe Pro Thr Ile Ser Ser Gly Pro Ile
145                 150                 155                 160

Asp Arg Tyr Arg Arg Tyr Lys Lys Asp Tyr Tyr Ser Val Pro Asn Lys
                165                 170                 175

Glu Lys Tyr Ile Gln Leu Leu Glu Lys Gly Leu His Tyr Ile Phe Leu
            180                 185                 190

Gly Phe Leu Tyr Asp Phe Met Leu Ser Tyr Phe Phe Gly Thr Val Leu
        195                 200                 205

Leu Pro Gly Ile Lys Arg Glu Val Ile Ala Ser Thr Gly Val Ser Leu
    210                 215                 220

Ala Leu Val Glu Tyr Met Tyr Val Tyr Ser Met Tyr Leu Phe Phe Asn
225                 230                 235                 240

Phe Ala Gly Tyr Ser Leu Phe Ala Val Gly Thr Ser Tyr Phe Met Gly
                245                 250                 255

Ile Glu Thr Pro Met Asn Phe Asn Gln Pro Phe Lys Ser Lys Asn Ile
            260                 265                 270

Lys Glu Phe Trp Asn Arg Trp His Met Thr Leu Ser Phe Trp Phe Arg
        275                 280                 285

Asp Tyr Val Tyr Met Arg Leu Val Phe Phe Phe Met Lys Lys Lys Val
    290                 295                 300

Phe Lys Asn Pro Lys Thr Thr Ala Asn Ile Thr Tyr Ile Leu Asn Met
305                 310                 315                 320

Leu Leu Met Gly Phe Trp His Gly Glu Thr Trp Tyr Tyr Ile Leu Tyr
                325                 330                 335

Gly Phe Ile His Gly Val Ala Leu Val Val Asn Asp Trp Trp Leu Gly
            340                 345                 350

Tyr Lys Lys Lys His Lys Asp Val Val Pro His Asn Lys Phe Thr Glu
        355                 360                 365

Leu Phe Ala Ile Phe Ile Thr Phe Asn Phe Val Cys Phe Thr Phe Leu

370                 375                 380

Ile Phe Ser Gly Ile Leu Asp Phe Val Lys Phe Arg
385                 390                 395

<210>  9
<211>  29
<212>  PRT
<213>  Lactobacillus salivarius

<400>  9

Gln Leu Asp Glu Glu Asp Cys Gly Ala Ala Val Leu Ala Met Ile Leu
1               5                   10                  15

Tyr Tyr Tyr Arg Ser Lys Ile Pro Met Ser Lys Ile Lys
            20                  25

<210>  10
<211>  18
<212>  PRT
<213>  Lactobacillus salivarius

<400>  10

His Tyr Leu Ile Ile Lys Lys Val Thr Ser Lys Tyr Val Glu Ile Val
1               5                   10                  15

Asp Pro

<210>  11
<211>  53
<212>  PRT
<213>  Lactobacillus salivarius

<400>  11

Met Phe Phe Asn Phe Met Lys Lys Val Asp Val Lys Lys Asn Phe Gly
1               5                   10                  15

Tyr Lys Glu Val Ser Arg Lys Asp Leu Ala Lys Val Asn Gly Gly Lys
            20                  25                  30

Arg Lys Lys His Arg Cys Arg Val Tyr Asn Asn Gly Met Pro Thr Gly
        35                  40                  45

Met Tyr Arg Trp Cys
    50

<210>  12
<211>  31
<212>  PRT
<213>  Lactobacillus salivarius

<400>  12

Met Phe Phe Asn Phe Met Lys Lys Val Asp Val Lys Lys Asn Phe Gly
1               5                   10                  15

Tyr Lys Glu Val Ser Arg Lys Asp Leu Ala Lys Val Asn Gly Gly
            20                  25                  30

<210>  13
<211>  162
<212>  DNA
<213>  Lactobacillus salivarius

<400>  13
atgtttttta attttatgaa aaaagtagat gtgaagaaga attttggata taaagaagtt      60

tctagaaaag atctagctaa agtaaatggt ggaaagagaa agaaacatcg ttgcagagtt     120

tataataatg gaatgcctac aggaatgtat cgttggtgct aa                        162

## Description

### Field of the Invention

This invention relates to an antimicrobial peptide having potent antibacterial activity against a range of bacteria, a nucleic acid encoding the antimicrobial peptide, a strain of *Lactobacillus salivarius* bacteria capable of expressing the antimicrobial peptide, an immunity protein conferring resistance to the antimicrobial effects of the antimicrobial peptide and a nucleic acid encoding the cognate immunity protein, and recombinant bacteria transformed with a nucleic acid encoding the antimicrobial peptide and/or a nucleic acid encoding the cognate immunity protein.

### Background to the Invention

The genus *Lactobacillus* contains in excess of 145 species whose habitats range from the extremities of soil and plants to the mammalian gastrointestinal tract (GIT). These bacteria are renowned for the rich diversity of antimicrobial peptides (bacteriocins) they produce, each of which is assigned a name specific to the individual producing member species. Additionally, these antimicrobial peptides show great diversity with respect to structure and mode of action varying from extensively post-translationally modified lantibiotics such as plantaricin C to large unmodified heat labile proteins such as helveticin.

*Lactobacillus salivarius* is a species particularly associated with the mammalian GIT, and has a number of associated probiotic attributes. Several studies have revealed favourable immunomodulatory properties of certain *L. salivarius* strains. The human ileal isolate *L. salivarius* UCC118 as well as *L. salivarius* CECT 5713, isolated from a mother and child pair, were both found to reduce colonic inflammation in animal models of colitis reducing the levels of pro-inflammatory cytokines such as TNF-α and IL-12. Likewise, *L. salivarius*-mediated stimulation of the anti-inflammatory cytokine IL-10 reduced lipopolysaccharide (LPS)-induced inflammatory responses in murine bone-marrow-derived macrophages *in vitro.* Interestingly, oral administration of *L. salivarius* UCC118 was also reported to reduce tumour development and mortality in IL-10 knockout mice. Pre-treatment of intestinal epithelial cells with *L. salivarius* UCC118 was found to significantly reduce *Salmonella typhimurim*-induced pro-inflammatory responses (IL-8 production) *in vitro.* In addition, a strain of *L. salivarius* was among six select strains chosen to formulate a multi-species probiotic for combating disease in critically ill patients due to its positive immunomodulatory and potent antimicrobial activity. The ability of a *L. salivarius* component of another five-strain probiotic combination, *L. salivarius* DPC6005, to dominate over four co-administered strains within the porcine ileal digesta and mucosa. This strain produced a potent antimicrobial activity of due to production of the antimicrobial peptide salivaricin P.

*L. salivarius* produces a range of antimicrobial compounds which have exhibited inhibitory activity toward several important gastrointestinal pathogens. Indeed, acid production mediated by *L. salivarius* induced reductions in the prevalence of peridontopathic pathogens in the oral cavity. Indeed, the corresponding strains were purported to be effective for the probiotic treatment of periodontal diseases. Strain-specific inhibition of *Helicobacter pylori* was also partly ascribed to acid production by inhibitory *L. salivarius* strains. The potent activity of *L. salivarius* CECT 5713 was ascribed to a combination of factors including acid and hydrogen peroxide production. This strain was also capable of inducing mucin production in HT29 cells *in vitro.* Adherence to hog mucin and co-aggregation with *Salmonella* (thereby reducing adhesion of the pathogen to mucin), was also demonstrated by strain CECT 5713 and likely contributed to the protective effect of this strain against *Salmonella* infection in a murine model. Furthermore, anti-staphylococcal activity associated with strain CECT 5713 may suggest its use as a preferential alternative to conventional antibiotics in the treatment of infectious mastitis in women during lactation. This effect was also a purported consequence of acid and hydrogen peroxide production. However, the presence of the genetic determinants for production of the abp 118 antimicrobial peptide were recently revealed in the genome of *L. salivarius* CECT 5713, the expression of which may also be a contributing factor to the potent antimicrobial activity of this strain. Recent safety assessments of strain CECT 5713 in an animal model, six-month old infants and healthy adults confirmed the beneficial immune modulatory properties and the safety of this strain for human consumption.

The range of characterized antimicrobial peptides produced by *L. salivarius* extends from the class IIa pediocin-like antimicrobial peptide OR-7 to two-component class IIb antimicrobial peptides, abp118, salivaricin P and variants thereof, as well as the recently described salivaricin T and class IId linear non-pediocin-like antimicrobial peptides such as salivaricin B. Each of these antimicrobial peptides has demonstrated inhibitory activity towards gastrointestinal or urogenital pathogens. Significantly, *L. salivarius* strains NRRL B-30514 and UCC118 have demonstrated OR-7- and abp118-mediated *in vivo* protection against *Campylobacter jejuni* and *Listeria monocytogenes* infection, respectively.

It is an object of the present invention to provide alternative antimicrobial peptides which have broad host range and which can function as a probiotic property.

### Summary of the Invention

Broadly, this invention provides a novel broad spectrum antimicrobial peptide, designated Bactofensin LS1, with potent anti-*L*. *monocytogenes* and anti-*S*. *aureus* activity which has been identified in *L. salivarius.* Genomic sequence analysis of the producing strain revealed the genetic determinants responsible for antimicrobial peptide production. The antimicrobial peptide locus encodes a highly basic antimicrobial peptide and an extremely unusual immunity system, and in this respect Bactofensin LS1 bears a closer resemblance to certain eukaryotic cationic antimicrobial peptides than to bacteriocins produced by bacteria. Indeed, the amino acid sequence of the cationic antimicrobial peptide did not display significant homology with previously characterized bacteriocins. In addition, Bactofensin LS1 displayed antimicrobial activity at micromolar concentrations, as do other eukaryotic antimicrobial peptides.

According to the invention, there is provided an isolated *Lactobacillus salivarius* DPC6502 strain as deposited with the National Collection of Industrial and Marine Bacteria under the Accession No. NCIMB 41840 on 9 May 2011 (deposited in the name of Teagasc Food Research Centre, Moorepark, Fermoy, Co. Cork, Ireland), and variants thereof, wherein the variants retain the phenotypic characteristics of the isolated bacteria, and wherein the isolated bacteria and variants thereof express an antimicrobial peptide having potent antibacterial activity against *Listeria monocytogenes* and *Staphylococcus aureus.* The isolated *Lactobacillus salivarius* DPC6502 strain, and variants thereof as defined above, are hereafter referred to as an "isolated strain of the invention".

Preferably, the antimicrobial peptide is the protein of SEQ ID NO: 2 (designated Bactofensin LS1), or a variant thereof having at least 90% sequence identity to SEQ ID NO: 2, and wherein the antimicrobial peptide of SEQ ID NO: 2, or the variant thereof, have potent antibacterial activity against *Listeria monocytogenes* and *Staphylococcus aureus.*

The invention further relates to an antimicrobial peptide of SEQ ID NO: 2 (Bactofensin LS1), or a variant thereof having at least 90% sequence identity with SEQ ID NO: 2, and wherein the antimicrobial peptide of SEQ ID NO: 2, or the variant thereof, have potent antibacterial activity against *Listeria monocytogenes* and *Staphylococcus aureus.* The antimicrobial peptide of SEQ ID NO: 2, and variants thereof as defined above, are hereafter referred to as "antimicrobial peptide of the invention".

Ideally, the antimicrobial peptide of the invention has an antibacterial activity against *Listeria monocytogenes* and *Staphylococcus aureus* of at least 40µM, 30µM, 20µM, 10µM, 9µM, 8µM, 7µM, 6µM or 5µM.

The invention also provides an isolated antimicrobial peptide having potent antibacterial activity against *Listeria monocytogenes* and *Staphylococcus aureus,* produced by an isolated strain of the invention, wherein the variant retains the phenotypic characteristics of the isolated bacteria, and expresses an antimicrobial peptide having potent antibacterial activity against *Listeria monocytogenes* and *Staphylococcus aureus.*

The invention also relates to a nucleic acid encoding the antimicrobial peptide of the invention.

The invention also relates to a nucleic acid having a sequence of SEQ ID NO: 1, or a variant thereof having at least 90% sequence identity with SEQ ID NO: 1, and wherein the nucleic acid of SEQ ID NO: 1, or the variant thereof, encode an antimicrobial peptide having potent antibacterial activity against *Listeria monocytogenes* and *Staphylococcus aureus.* The nucleic acid of SEQ ID NO: 1, and variants thereof as defined above, are hereafter referred to as "nucleic acid of the invention".

The Applicants have also surprisingly discovered a gene in *Lactobacillus salivarius* DPC6502 strain which encodes a protein that confers immunity on the host strain to the antibacterial effects of the antimicrobial peptide of the invention (termed "cognate immunity protein"). Thus, the invention also relates to an isolated protein having an amino acid sequence of SEQ ID NO: 8, or an isolated variant thereof comprising a sequence having at least 90% sequence identity with SEQ ID NO: 8, wherein the isolated protein or variant thereof is capable of conferring immunity on a host strain, typically a *Lactobacillus* host strain, ideally a *Lactobacillus salivarius* host strain, to the antibacterial effects of the antimicrobial peptide of the invention. The isolated protein of SEQ ID NO: 8, and isolated variants thereof, are hereafter referred to as "cognate immunity proteins of the invention".

The invention also relates to a nucleic acid encoding a cognate immunity protein of the invention, for example a nucleic acid comprising a nucleic acid of SEQ ID NO: 7.

The invention also provides a recombinant vector comprising one or more of: a nucleic acid of the invention; a nucleic acid encoding an antimicrobial peptide of the invention; and a nucleic acid encoding a cognate immunity protein of the invention. In a particularly preferred embodiment of the invention, the recombinant vector comprises a nucleic acid encoding an antimicrobial peptide of the invention (for example a nucleic acid of SEQ ID NO: 1 or a nucleic acid encoding an antimicrobial peptide of SEQ ID NO: 2); and a nucleic acid of SEQ ID NO 7 or a nucleic acid encoding a cognate immunity protein of the invention (for example a nucleic acid encoding a protein of SEQ ID NO: 8);

The invention also relates to a host cell transformed by a recombinant vector of the invention (hereafter "host cell of the invention").

The invention also relates to an antimicrobial peptide of the invention for use as a medicament.

The invention also relates to an antimicrobial peptide of the invention for use as an antibacterial agent or an antibiotic.

The invention also relates to an antimicrobial peptide of the invention for use in treating or preventing a disease or condition characterised by growth of *Listeria* or *Staphylococcus aureus.*

The invention also relates to a pharmaceutical composition comprising an antimicrobial peptide of the invention in combination with a suitable pharmaceutical excipient.

The invention also relates to an antibacterial or antibiotic formulation comprising the antimicrobial peptide of the invention.

The invention also relates to an isolated strain of the invention, or a transformed host cell, of the invention, for use as a probiotic culture.

The invention also relates to an isolated strain of the invention, or an antimicrobial peptide of the invention, for use as a biopreservative, for use in treating microbial infections and clinical infections, for use as a disinfectant, for use as an antibiotic in animal husbandry applications, and for use in reducing the incidence of sensitive methicillin-resistant *S*. *aureus* in animals, especially pigs.

### Brief Description of the Drawings

The invention will be more clearly understood from the following description of an embodiment thereof, given by way of example only, with reference to the accompanying drawings, in which:-
Figure 1 illustrates a HPLC profile (A), MALDI-TOF MS data (B), and antimicrobial activity (C) of purified Bactofensin LS1;
Figure 2 illustrates the nucleotide sequence (SEQ ID NO. 1) and deduced pro-peptide sequence (SEQ ID NO. 2) of mature Bactofensin LS1 peptide, together with the precursor peptide (SEQ ID NO. 11), leader sequence (SEQ ID NO. 12), and full nucleic acid sequence (SEQ ID NO. 13) of the structural gene of Bactofensin LS1. The leader sequence is underlined and the GG-processing site is indicated by a bold triangle (▲);
Figure 3 illustrates the nucleotide sequence (SEQ ID NO. 7) and the deduced peptide sequence (SEQ ID NO. 8) of the cognate immunity gene of Bactofensin LS1 (See also Table 2); and

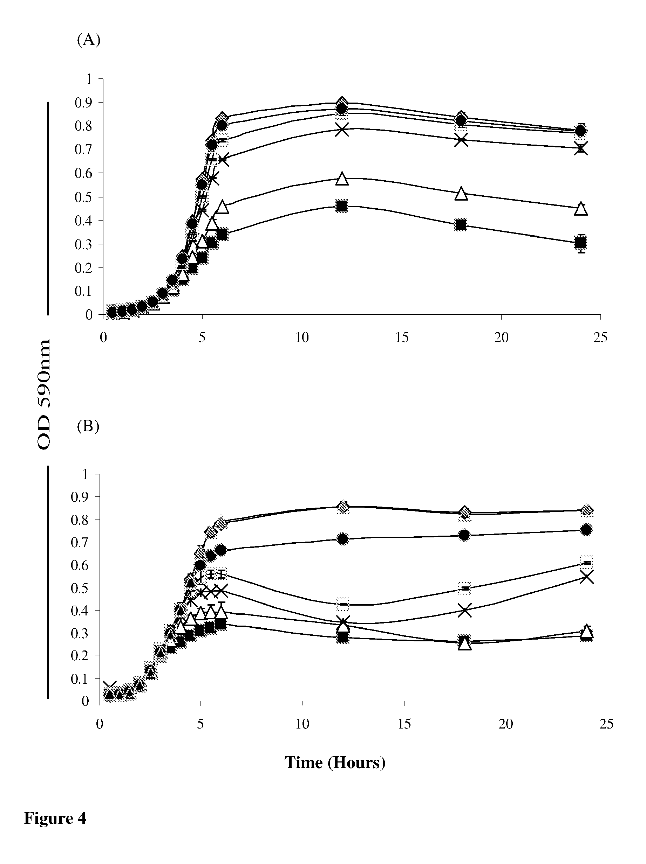

Figure 4 illustrates a graph demonstrating the inhibitory effect of synthetic Bactofensin LS1 on the growth of the indicator strains *Listeria monocytogenes* NCTC 11994 (A), and *Staphylococcus aureus* DPC5246 (B) at concentrations of 0 µM (◆), 0.05 µM (Δ), 0.1 µM (●), 0.5 µM (□), 1.0 µM (×), 5.0 µM (Δ) and 10.0 µM (■). Error bars represent standard deviations based on triplicate data.

### Detailed Description of the Drawings

Broadly, the invention is based on the discovery of an antimicrobial peptide designated Bactofensin LS1 which has potent antibacterial activity against a broad spectrum of bacteria, including the pathogenic *Listeria monocytogenes and Staphylococcus aureus* strains. The amino acid sequence of Bactofensin LS1 is provided in SEQ ID NO: 2. A source of Bactofensin LS1 is a strain of *Lactobacillus salivarius* that was isolated from porcine jejunum bacterial isolates, designated *Lactobacillus salivarius* DPC6502, which has been deposited with the National Collection of Industrial and Marine Bacteria under the Accession No. NCIMB 41840 on 9 May 2011 (in the name of Teagasc Food Research Centre, Moorepark, Fermoy, Co. Cork, Ireland). The applicants have also discovered a protein expressed by *Lactobacillus salivarius* DPC6502 that confers immunity on its host against the antibacterial effects of Bactofensin LS1. This protein, designed an "immunity protein" has an amino acid sequence provided in SEQ ID NO: 8.

The term "variant" as applied to *Lactobacillus salivarius* DPC6502 should be understood to mean strains of *Lactobacillus salivarius* that retain the phenotypic characteristics of the DPC6502 strain and which express an antimicrobial peptide having potent antibacterial activity against *Listeria monocytogenes* and *Staphylococcus aureus,* typically an antimicrobial peptide of SEQ ID NO: 2 or a variant thereof. Examples of these variant strains include *L. salivarius* DPC6005, DPC6027, DPC6189 and 7.3. In one preferred embodiment, the term "variant" excludes *L. Salivarius* DPC6005. In one embodiment, the term variants should be understood to mean progeny (unmodified descendents), modified descendents, or derivatives of *Lactobacillus salivarius* DPC6502, for example strains which are genetically modified to alter the genotype of the bacteria, or strains which are altered by natural processes such as selection.

The term "phenotypic characteristics" as applied to the isolated bacteria *Lactobacillus salivarius* DPC6502 as deposited with the National Collection of Industrial and Marine Bacteria under the Accession No. NCIMB 41840 should be understood to mean a bacterium isolated from the intestinal region, typically mammalian intestine such as human or porcine intestine, typically the porcine jejunum, and which is capable of expressing an antimicrobial peptide that has potent antibacterial activity against *Listeria, Staphylococcus aureus,* and ideally at least two *Lactobacillus* strains.

The term "variants" as applied to the antimicrobial peptide of SEQ ID NO: 2 should be understood to mean peptides comprising a sequence having at least 90% sequence identity with SEQ ID NO: 2, and have potent antibacterial activity against *Listeria monocytogenes* and *Staphylococcus aureus.* The peptides will generally have less than 100 residues, 60 residues, 50 residues, 40 residues, 30 residues or 25 residues, and can include immature forms of the peptide of SEQ ID NO: 2, for example a precursor peptide such as in SEQ ID NO. 11. Suitably, the variants consist of, or comprise, a sequence having at least 91%, 92%, 93%, 94%, 95%, 96%, 97%, 98% or 99% sequence identity with SEQ ID NO: 2. Variants of the antimicrobial peptide of SEQ ID NO: 2 shall preferably be taken to mean peptides having amino acid sequences which are substantially identical to SEQ ID NO: 2. Thus, for example, the term should be taken to include proteins or polypeptides that are altered in respect of one or more amino acid residues, for example 1, 2, 3, 4, 5, 6, 7, 8, 9, or 10 amino acids. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Typically, Bactofensin LS1 proteins of the invention which have been altered by substitution or deletion of residues that are critical to its antibacterial activity will be excluded from the term "variant". The term sequence identity comprises both sequence identity and similarity, i.e. a polypeptide sequence that shares 90% amino acid identity with SEQ ID NO: 2 is one in which any 90% of aligned residues are either identical to, or conservative substitutions of, the corresponding residues in SEQ ID NO: 2. The term "variant" is also intended to include chemical derivatives of Bactofensin LS1 protein, *i.e.* where one or more residues of Bactofensin LS1 is chemically derivatized by reaction of a functional side group. Also included within the term variant are Bactofensin LS1 molecules in which naturally occurring amino acid residues are replaced with amino acid analogues. Details of amino acid analogues will be well known to those skilled in the art.

Proteins and polypeptides (including variants and fragments thereof) of and for use in the invention may be generated wholly or partly by chemical synthesis or by expression from nucleic acid. The proteins and peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984)).

The term "variant" as applied to a nucleic acid of SEQ ID NO: 1 (the nucleic acid encoding the antimicrobial peptide of SEQ ID NO: 2) should be understood to mean a nucleic acid that includes a sequence having at least 90% sequence identity with SEQ ID NO: 1, and which encode an antimicrobial peptide having potent antibacterial activity against *Listeria monocytogenes* and *Staphylococcus aureus.* The term should also be understood to mean nucleic acids that encode a variant antimicrobial peptide of the invention. Suitably, the variants have at least 91%, 92%, 93%, 94%, 95%, 96%, 97%, 98% or 99% sequence identity with SEQ ID NO: 1. The term also includes nucleic acids encoding the precursor peptide of SEQ ID NO. 11, for example, the nucleic acid of SEQ ID NO. 13.

The invention also relates to an isolated immunity protein of the invention. This term should be understood to mean a protein of SEQ ID NO: 8, or a variant thereof which is capable of conferring immunity on a host strain, typically a *Lactobacillus* host strain, ideally a *Lactobacillus salivarius* host strain, to the antibacterial effects of the antimicrobial peptide of the invention. Thus, a bacteria which expresses an immunity protein of the invention will be immune to the antibacterial effects of the antimicrobial peptide of the invention, or will have increased immunity compared to a bacteria which does not express the immunity protein of the invention. Suitably, the variants comprise a sequence having at least 90%, 91%, 92%, 93%, 94%, 95%, 96%, 97%, 98% or 99% sequence identity with SEQ ID NO: 8.

The invention also relates to a recombinant vector comprising a nucleic acid encoding an antimicrobial peptide of the invention, a nucleic acid encoding a cognate immunity protein of the invention, or one or more nucleic acids encoding an antimicrobial peptide and a cognate immunity protein of the invention. The nucleic acids may be cloned as separate entities (i.e. distinct nucleic acid constructs), or in a same construct, under distinct promoter regions or in a single operon. Typically, the nucleic acids are cloned into a recombinant vector (for example a plasmid) which is capable of replicating in the host bacteria. Typical plasmids contain, in addition to the cloned insert, a selection gene (i.e. antibiotic resistance, a dye etc) and an origin of replication effective in the host bacterium. The plasmid may also comprise regulatory sequences, for example promoters, terminators and/or enhancers. Examples of such vectors are pNZ44 (McGrath S, Fitzgerald GF, van Sinderen D (2001) Improvement and optimization of two engineered phage resistance mechanisms in Lactococcus lactis. Appl. Environ. Microbiol. 67 (2): 608-616)) and pCI372 (Hayes F, Daly C, Fitzgerald GF (1990) Identification of the Minimal Replicon of Lactococcus lactis subsp. lactis UC317 Plasmid pCI305. Appl. Environ. Microbiol. 56: 202-209)). However, any recombinant vector suitable for replicating in a host bacteria known to the person skilled in the art may be used.

The nucleic acid may also be cloned into an integrative cassette suitable for integration into the genome of suitable host bacteria. Such an integrative cassette typically comprises a nucleic acid encoding an antimicrobial peptide of the invention or a cognate immunity protein of the invention, or both, linked to (or flanked by) one or several sequences allowing integration, preferably site-specific integration. Such sequences may be for instance nucleic acid sequences homologous to a targeted region of the genome, allowing integration through crossing over. Various techniques can be used to insert a nucleic acid into a host bacteria, for example through natural transformation or electroporation.

The host bacteria suitable for cloning the antimicrobial peptide and/or the cognate immunity protein may be selected from any host bacteria known to a person skilled in the art such as, for example, *Lactococcus, Lactobacillus and Enterococcus.*

In this regard, the term "animal husbandry-related applications" should be understood to mean that the antimicrobial peptide can be used for reducing the incidence of *S. aureus* or for the treatment of *Staphylococcus aureus*-related infections in animals such as for example mastitis, or as a probiotic trait for producing strains in animal feed applications (the antimicrobial peptide should contribute to the dominance of producing strains in the intestine by killing competing flora).

In the specification, the term "potent antibacterial activity against *Listeria monocytogenes* and *Staphylococcus aureus"* as applied to the antimicrobial peptides (AMPs) of the invention should be understood to mean that a concentration of AMP in the range of 0.1µM to 50µM is sufficient to inhibit the growth of both *S*. *aureus* DPC5246 and *L. Monocytogenes* NCTC11994 by 50% using the broth-based MIC50 assay described below. In this specification, the term "antimicrobial activity of at least XµM against *Listeria monocytogenes* and *Staphylococcus aureus"* as applied to the antimicrobial peptides (AMPs) of the invention should be understood to mean that a concentration of AMP of at most XµM is sufficient to inhibit the growth of both S. *aureus* DPC5246 and *L. Monocytogenes* NCTC11994 by 50% using the broth-based MIC50 assay described below.

In one embodiment, the term "cognate immunity protein" should be understood to mean a protein, which, when expressed, increases strain resistance to the antimicrobial peptide by increasing the content of esterified D-alanyl groups of techoic acids on the bacterial cell surface thereby resulting in a decrease in the net negative charge of the bacterial cell wall and reducing the potential of the initial electrostatic interaction of the cell with the cationic antimicrobial peptide.

### Material and Methods

### Bacterial strains and culture conditions.

Indicator strains employed for antimicrobial characterization included *Escherichia coli* DH5α, nontoxigenic *E. coli* O157:H7 strain P1432, *Enterobacter sakazakii* NCTC08155, *Listeria innocua* DPC3572, *Listeria monocytogenes* NCTC11994, *Staphylococcus aureus* DPC5246, and methicillin-resistant *S*. *aureus* (MRSA) DPC5646, all of which were grown aerobically at 37°C in BHI (Merck, Darmstadt, Germany). *L. salivarius* strains used in this study are listed in Table 1 below. All lactobacilli were routinely cultured under anaerobic conditions at 37°C in MRS medium (Difco Laboratories, Detroit, MI), unless otherwise stated. Anaerobic conditions were maintained with the use of anaerobic jars and anaerocult A gas packs (Merck).

**Table 1. Lactobacillus salivarius strains used in this study.**

| **Strain** | **Relevant features** | **Reference** |
|---|---|---|
| *Lactobacillus salivarius* UCC118 | Abp118 producer | Flynn *et al.,* 2002 |
| *Lactobacillus salivarius* DPC6502 | Bactofensin LS1 producer | O'Shea *et al.,* 2009 |
| *Lactobacillus salivarius* DPC6488 | Salivaricin T producer | O'Shea *et al.,* 2009 |
| *Lactobacillus salivarius* DPC6005 | Salivaricin P producer; bactofensin LS1 producer | Barrett *et al.,* 2007 |
| *Lactobacillus salivarius* 7.3 | Salivaricin P producer; bactofensin LS1 producer | Barrett *et al.,* 2007 |
| *Lactobacillus salivarius* DPC6189 | Salivaricin P producer; bactofensin LS1 producer | Barrett *et al.,* 2007 |
| *Lactobacillus salivarius* DPC6027 | Salivaricin P producer; bactofensin LS1 producer | Barrett *et al.,* 2007 |
| *Lactobacillus salivarius* DPC6196 | Bac despite harbouring bacteriocin structural genes | Barrett *et al.,* 2007 |

(Barrett, E., Hayes, M., O'Connor, P., Gardiner, G., Fitzgerald, G., Stanton, C., Ross, R.P. and Hill, C., (2007) Salivaricin P: one of a family of two component anti-listerial antimicrobial peptides produced by intestinal isolates of Lactobacillus salivarius. Appl. Environ. Microbiol. 73: 3719-3723; Flynn, A., van Sinderen, D., Thornton, G. M., Holo, H., Nes, I. F., and Collins, J. K., (2002) Characterization of the genetic locus responsible for the production of ABP-118, a novel antimicrobial peptide produced by the probiotic bacterium Lactobacillus salivarius subsp. salivarius UCC118. Microbiology 148: 973-984.)

### Purification of the hydrophilic antimicrobial peptide produced by L. salivarius DPC6502.

The antimicrobial peptide was purified from a 0.5 L overnight culture of *L. salivarius* DPC6502 grown in MRS-IM-G media. The cells were removed by centrifugation at 8, 000 × g for 15 min, and the supernatant applied to a column containing 10 ml SP sepharose Fast flow cation-exchange resin (GE Healthcare, UK) previously equilibrated with 50 mM sodium acetate buffer, pH 4.5. The column was washed with 50 mM sodium acetate buffer, pH 4.5 containing 0.3 M NaCl, the bioactive peptide was subsequently eluted from the column using 50 mM sodium acetate buffer, pH 4.5 containing 1.0 M NaCl. The eluate was applied to a 2.0 g (12 ml volume) C₁₈ Bond Elute column (Phenomenex, Cheshire, UK) pre-equilibrated with methanol and water. The column was then washed with 0.1% (vol/vol) trifluoroacetic acid (TFA) and the bioactive peptide eluted with 70% (vol/vol) propan-2-ol containing 0.1% (vol/vol) TFA. The propan-2-ol was removed by rotary evaporation and 250µl aliquots of the resultant preparation were applied to a Jupiter proteo reversed-phase high-performance liquid chromatography (RP-HPLC) column (250.0 × 4.6 mm, 4 µm particle size, 90 Ǻ pore size; Phenomenex). The column was pre-equilibrated with 10% acetonitrile containing 0.1% (vol/vol) TFA, followed by separation and elution of the bioactive peptide by gradient RP-HPLC using 0.1% (vol/vol) TFA and acetonitrile concentrations that ranged from 10% to 30% (vol/vol), from 5 to 45 min at a flow rate of 1.0 ml min⁻¹. Absorbance was monitored at a wavelength of 214 nm. Antimicrobial peptide activity was monitored throughout the purification procedure by well diffusion assay using the sensitive indicator strain *L. delbrueckii* subsp. *bulgaricus* LMG6901. Matrix-assisted laser desorption ionization-time of flight mass spectrometry (MALDI-TOF MS) (AXIMA-TOF²; ShimadzuBiotech, Manchester, UK) analysis was performed on bioactive fractions as described previously (Cotter, P.D., Deegan, L.H., Lawton, E.M., Draper, L.A., O'Connor, P.M., Hill, C. and Ross, R.P. (2006) Complete alanine scanning of the two-component lantibiotic lacticin 3147: generating a blueprint for rational drug design. Mol Microbiol, 62, 735-747). Fractions of interest were further purified by reapplying them to the RP-HPLC column under the conditions described above. N-terminal sequence analysis of the purified antimicrobial peptide by Edman degradation was performed by Aberdeen Proteomics (University of Aberdeen, Scotland). Reduction and alkylation of the cysteine residues was performed using dithiolthreitol (DTT) and iodoacetamide.

### Genome sequencing and analysis.

The sequence of the genomic DNA extracted from *L. salivarius* DPC6502 was determined through random shotgun pyrosequencing (Beckman Coulter Genomics, USA). The draft genome assembly was processed using the annotation software package GAMOLA. The gene model was determined with Gene Locator and Interpolated Markov ModelER 3.02 (GLIMMER). Sequence similarity analyses were performed using gapped BLASTp algorithm and the non-redundant database provided by the NCBI (ftp://ftp.ncbi.nih.gov/blast/db).

### Generation of a synthetic analogue of Bactofensin LS1.

The Bactofensin LS1 peptide was synthesized according to the deduced amino acid sequence of the chromosomally located antimicrobial peptide structural gene (DLSL_0050) using microwave-assisted solid-phase peptide synthesis (MW-SPPS) performed on a CEM Liberty^{™} microwave peptide synthesiser using a H-Cys-HMPB-ChemMatrix® resin (PCAS Biomatrix Inc., Quebec, Canada). The synthetic peptide was purified by RP-HPLC as described above. Fractions containing a peptide with the desired molecular mass, as identified by MALDI-TOF MS, were pooled and lyophilised using a Genevac HT 4X evaporator (Genevac Ltd. Ipswitch, United Kingdom). The peptide was dissolved in 70% (vol/vol) propan-2-ol at a concentration of 5 mg ml⁻¹ and stored at -20°C. Appropriate dilutions of the peptide in 50 mM sodium phosphate buffer, pH 6.8, were used to determine the specific activity of the synthetic analogue.

### Specific activity determination.

A microtiter plate assay system was used to determine the minimum concentration of the synthetic Bactofensin LS1 analogue required to inhibit the growth of a range of pathogenic indicator strains (Table 1), by 50% (MIC₅₀). The microtitre plate was first treated with bovine serum albumin (BSA) to prevent adherence of the peptide to the sides of the wells. Each plate included triplicate assays at each concentration of synthetic Bactofensin LS1 examined. Each well contained a total volume of 200 µl, comprised of purified Bactofensin LS1, and 150 µl of a 1-in-10 dilution of the indicator culture (*A*₅₉₀ of 0.1) in BHI broth. Control wells contained media only (blanks), and untreated indicator culture. The microtiter plate cultures were incubated at 37°C for 24 h, and the optical densities at 590 nm (OD₅₉₀) recorded at 30 min intervals (GENios plus; TECAN, Switzerland). Triplicate readings were averaged and blanks were subtracted from these readings. The amount of antimicrobial peptide that inhibited the indicator strain by 50% was defined as 50% of the final OD₅₉₀ ± 0.05 of the untreated control culture.

### Detection of salS.

The presence of *salS* was determined by PCR using template DNA from six additional genetically distinct intestinal *L. salivarius* isolates (Table 1) and the primer pair: SalSF 5' CAGTCGACAATGATCATGATGGAGTAGCG 3' (SEQ ID NO. 3) and SalSR 5' GGAAGTAAGTAGGGTTTTGATAAGGTGTC 3' (SEQ ID NO. 4) to amplify a product of 430 nucleotides. This was performed using Expand High Fidelity PCR system (Roche) according to the manufacturers' instructions. The products derived from PCR were purified and sequenced (Beckman coulter genomics), and analysis of DNA sequence data was performed using LASERGENE 6 software (DNAStar Inc., Madison, WI). Template DNA of *L. salivarius* DPC6502 and *L. salivarius* UCC118 were used as positive and negative controls, respectively.

### Results and Discussion

*L. salivarius* DPC6502 from porcine jejunal digesta was described previously (O'Shea *et al.,* 2009). This strain exhibited broad spectrum antimicrobial activity, while a false positive PCR result led to the incorrect characterization of this strain as an abp118-variant producer. Recent array comparative genomic hybridization (aCGH) analyses revealed the absence of abp118-related homologues in strain DPC6502. Indeed, this strain was the most divergent of seven *L. salivarius* test strains compared with the genome of the reference strain *L. salivarius* UCC118 (Claesson, M.J., Li, Y., Leahy, S., Canchaya, C., van Pijkeren, J.P., Cerdeno-Tarraga, A.M., Parkhill, J., Flynn, S., O'Sullivan, G.C., Collins, J.K., Higgins, D., Shanahan, F., Fitzgerald, G.F., van Sinderen, D. and O'Toole, P.W. (2006) Multireplicon genome architecture of Lactobacillus salivarius. Proc Natl Acad Sci U S A, 103, 6718-6723), exhibiting just 78% conservation of strain UCC118-specific gene content.

### Characterization of the antimicrobial phenotype of L. salivarius DPC6502.

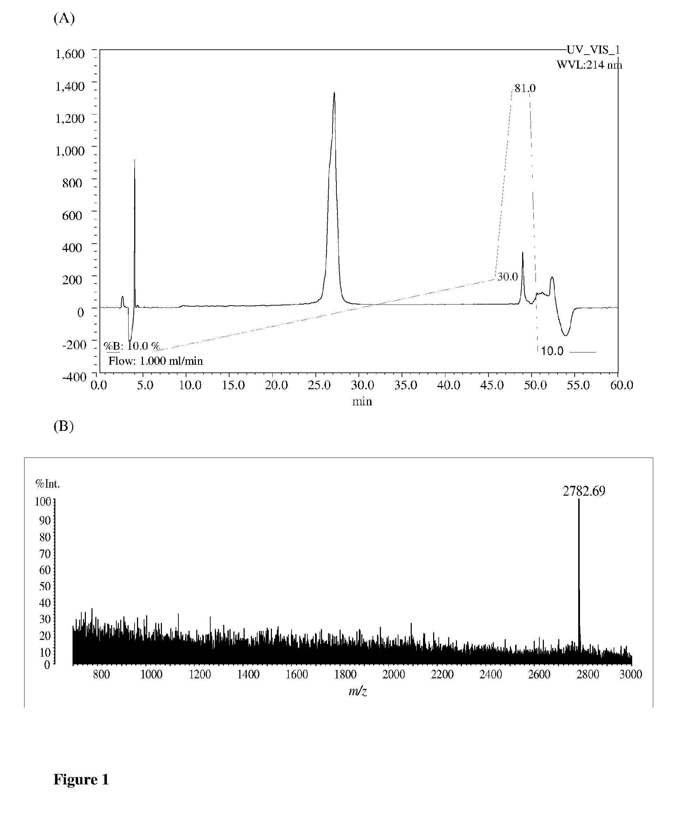

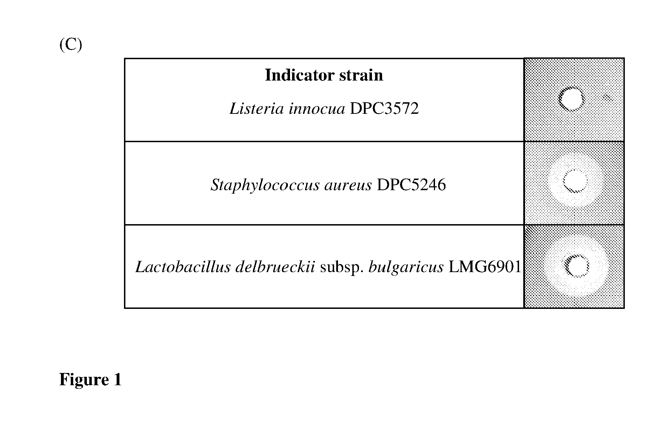

The peptide responsible for the antimicrobial activity of *L. salivarius* DPC6502 was purified from an overnight culture of the strain using cation-exchange chromatography and subsequent MALDI-TOF MS analysis revealed an associated mass of 2,785 Da in the active fractions (Figure 1). Edman analysis of the purified active fractions revealed the following N-terminal sequence: KRKXHRXRVYNNGMPTGMYRYM (SEQ ID NO. 5), where X at positions 4 and 7 indicate blank cycles for which no amino acid derivative was detected. A homology search did not identify any similar sequences in protein databases, indicating that this peptide is unlike any other previously characterized antimicrobial peptide and thus, was designated Bactofensin LS1. A previous assessment of the antimicrobial activity of this porcine isolate, using agar well diffusion assays with neutralised cell free supernatant (CFS), revealed a broad spectrum of inhibition which included 22 of 62 indicator strains (O'Shea et al., 2009). However, this activity was found to be predominantly against closely related strains of lactic acid bacteria (LAB). The availability of purified Bactofensin LS1 facilitated further investigations which established that the antimicrobial peptide is active against *L*. *delbrueckii* subsp. *bulgaricus, Listeria* and *Staphylococcus aureus* (Figure 1).

Comparative genomic analyses have previously revealed considerable intraspecies diversity in *L. salivarius.* A study of the relatedness between seven intestinal *L*. *salivarius* isolates, with an antimicrobial peptide-positive genotype, and the genome sequenced human probiotic strain *L. salivarius* UCC118 revealed that the Bactofensin LS1-producing porcine intestinal isolate DPC6502 differed most extensively from UCC118. As a consequence of this divergence, the fact that no porcine-derived *L*. *salivarius* isolates have been sequenced to date and the production of an apparently novel antimicrobial peptide, the genome of DPC6502 was targeted for genome sequencing.

### Characterization of the Bactofensin LS1 locus in the genome of L. salivarius DPC6502.

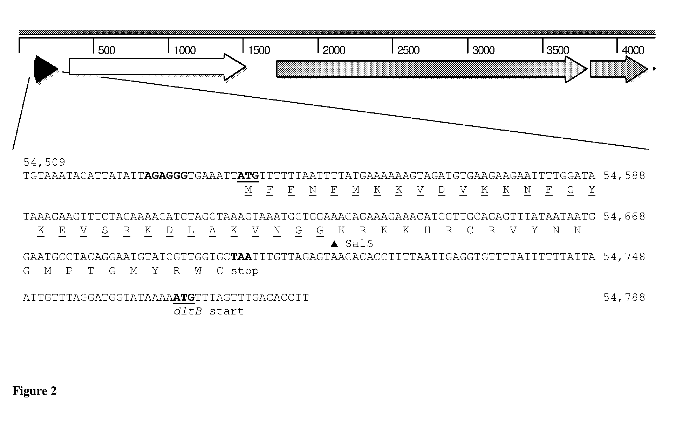

Scanning of the entire draft genome sequence for a gene corresponding to the amino acid sequence of Bactofensin LS1, as determined by Edman degradation, revealed a chromosomally located open reading frame (ORF), DLSL_0050, present on DPC6502-specific gene cluster 1 which was designated *bfls1.* The unidentified amino acids (aa) at positions 4 and 7 in the sequence determined by Edman degradation were, on the basis of the corresponding codons, found to be lysine and cysteine residues respectively, as shown in Figure 2. The *bfls1* structural gene consists of 162 nucleotides (SEQ ID No: 1) and is predicted to encode a 53 aa precursor peptide (SEQ ID NO. 11) comprised of a 31 aa double-glycine leader sequence (SEQ ID NO. 12) and a 22 aa propeptide sequence (SEQ ID No: 2) which differed from that derived by peptide sequencing with respect to the two most C-terminally located residues (Figure 2). The leader sequence of the antimicrobial peptide pre-peptide is unusually long (31 aa), nevertheless, it conforms with the characteristic consensus sequence of double-glycine leaders-**VS**RK**DL**AK**V**N**GG** (as highlighted in bold font - SEQ ID No. 6). The predicted mass of the mature translation product (2,785 Da) differed from that determined by MALDI-TOF MS analysis of the active peptide (2,782 Da) by 3 Da. MS analysis revealed an increase of 2 Da in the mass of the active peptide when the cysteine residues were assumed to be in a reduced state (after treatment with DTT and iodoacetamide; 2,784 Da), indicating that an intramolecular disulfide bond is formed between Cys7 and Cys22. The fact that bioactivity did not require extensive post-translational modification of the peptide and the absence of the characteristic pediocin-like consensus motif (YGNGV) of class IIa antimicrobial peptides suggests that this peptide belongs to the diverse class IId antimicrobial peptides of Gram-positive bacteria.

A database search did not identify any homologues for this ORF, indicating that DPC6502 produces a novel antimicrobial peptide, designated Bactofensin LS1. The mature Bactofensin LS1 peptide (SEQ ID No. 2) is highly basic containing eight positively charged residues, largely concentrated at the N-terminus (Figure 2), which may be involved in mediating the initial binding of the antimicrobial peptide to target cells via electrostatic interaction. Database searches have revealed that this peptide does not share significant homology with previously characterized antimicrobial peptides but perhaps more closely resembles eukaryotic antimicrobial peptides. Indeed a search of the antimicrobial peptides database revealed that Bactofensin LS1 shares greatest similarity (42%) with the plant antimicrobial peptide Ib-AMP3 isolated from extracts of the seed of *Impatiens balsamina* which displays both antibacterial and antifungal properties.

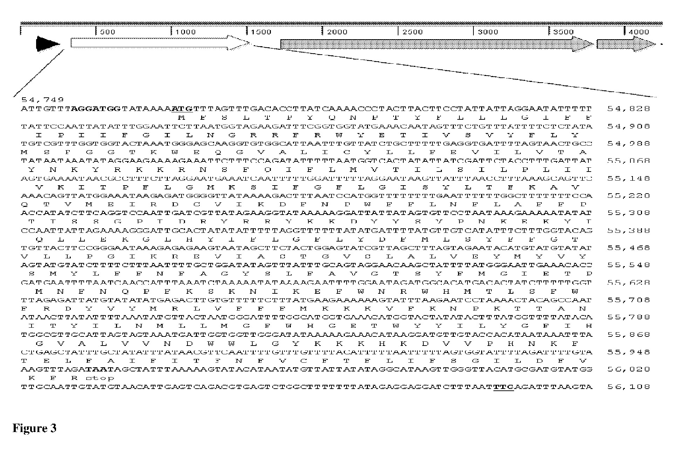

Typically, class II antimicrobial peptide structural genes are co-transcribed with an ORF encoding a cognate immunity protein located downstream of the structural gene to provide self-protection for the producing strain. The deduced 396 aa product (SEQ ID NO: 8 (see Figure 3 and Table 2 below)) of the ORF located immediately downstream of the antimicrobial peptide structural gene is uncharacteristically large for antimicrobial peptide immunity proteins. Moreover, this gene product shares 74% identity with an operon-encoded D-alanyl transfer protein (DltB) of *Pediociccus pentosaceus* ATCC25745 (Accession No. YP_805052) and 65% identity with the operon-encoded DltB of *L. salivarius* UCC118 (LSL_0976). Generally, *dltB* is located within the dlt operon, which is responsible for the D-alanylation of teichoic acids on the bacterial cell wall. Teichoic acids are predominantly negatively charged, and consequently, are a major determinant of the cell wall electrostatic interactions. D-alanylation of teichoic acids has previously been implicated in bacterial resistance to cationic antimicrobial peptides due to the resultant reduction in the net negative charge of the bacterial cell wall. DItB is a transmembrane protein responsible for the transfer of activated D-alanine across the cytoplasmic membrane which is indispensable for the D-alanyl esterification of teichoic acids. Another gene present on a *dlt* operon (DLSL_0974-DLSL_0978) is also located on the DPC6502 chromosome, the product of which shares 99% identity with DltB of UCC118 (encoded by LSL_0891) and 65% ID with the deduced protein product of DLSL_0051. The ORFs downstream of the *dltB* homologue encode a putative antimicrobial peptide ABC-transporter (DLSL_0052) and an antimicrobial peptide transport accessory protein (DLSL_0053). The deduced protein sequence of the ABC transporter contains an N-terminal peptidase C39 domain of 139 amino acids in length which contains a conserved cysteine motif (**Q**LDEE**DCG**A**A**V**L**A**MI**LYY**Y**RSKIP**M**SK**IK** - SEQ ID NO. 9) and histidine motif (**HYLII**KKVTSKYVE**I**V**DP** - SEQ ID No. 10) characteristic of the putative catalytic site responsible for the cleavage of double-glycine leader sequences. Thus, these genes encode the proteins which are probably responsible for the processing and secretion of mature active Bactofensin LS1. This putative antimicrobial peptide transport system likely completes the antimicrobial peptide gene cluster (approximately 4kb) as the adjacent ORFs, which are conserved in UCC118 (LSL_0035-LSL_0042), display similarity to the WalRK (YycGF) regulon responsible for the regulation of bacterial cell wall metabolism of low G + C Gram-positive bacteria.

**Table 2**

| |
|---|
| ***SEQ ID NO 7:** nucleic acid sequence of bactofensin LSI immunity gene (1191 nt)* |
| ATGTTTAGTTTGACACCTTATCAAAACCCTACTTACTTCCTATTATTAGGAATATTTTTTATTCCAATTA TATTTGGAATTCTTAATGGTAGAAGATTTCGGTGGTATGAAACAATAGTTTCTGTTTATTTTCTCTATAT GTCGTTTGGTGGTACTAAATGGGAGCAAGGTGTGGCATTAATTTGTTATCTGCTTTTTGAGGTGATTTTA GTAACTGCCTATAATAAATATAGGAAGAAAAGAAATTCTTTCCAGATATTTTTAATGGTCACTATATTAT CGATTCTACCTTTGATTATAGTGAAAATAACGCCTTTCTTAGGAATGAAATCAATTTTTGGATTTTTAGG AATAAGTTATTTAACCTTTAAAGCAGTTCAAACAGTTATGGAAATAAGAGATGGGGTTATAAAAGACTTT AATCCATGGTTTTTTTTGAATTTTTTGGCTTTTTTTCCAACCATATCTTCAGGTCCAATTGATCGTTATA GAAGGTATAAAAAGGATTATTATAGTGTTCCTAATAAAGAAAAATATATCCAATTATTAGAAAAGGATT GCACTATATATTTTTAGGTTTTTTATATGATTTTATGTTGTCATATTTCTTTGGTACAGTGTTACTTCCG GGAATAAAGAGAGAAGTAATAGCTTCTACTGGAGTATCGTTAGCTTTAGTAGAATACATGTATGTATATA GTATGTATCTTTTCTTTAATTTTGCTGGATATAGTTTATTTGCAGTAGGAACAAGCTATTTTATGGGAAT TGAAACACCGATGAATTTTAATCAACCATTTAAATCTAAAAATATAAAAGAATTTTGGAATAGATGGCAC ATGACACTATCTTTTTGGTTTAGAGATTATGTATATATGAGACTTGTGTTTTTCTTTATGAAGAAAAAAG TATTTAAGAATCCTAAAACTACAGCCAATATAACTTATATTTAAATATGTTACTAATGGGATTTTGGCA TGGTGAAACATGGTACTATATACTTTATGGTTTTATACATGGCGTTGCATTAGTAGTAAATGATTGGTGG TTGGGATATAAAAAGAAACATAAGGATGTTGTACCACATAATAAATTTACTGAGCTATTTGCTATATTTA TAACGTTCAATTTTGTTTGTTTTACATTTTTAATTTTTAGTGGTATTTTAGATTTTGTAAAGTTTAGATA A |
| ***SEQ ID NO 8:** amino acid sequence of bactofensin LSI immunity protein (396 aa)* |
| MFSLTPYQNPTYFLLLGIFFIPIIFGILNGRRFRWYETIVSVYFLYMSFGGTKWEQGVALICYLLFEVIL VTAYNKYRKKRNSFQIFLMVTILSILPLIIVKITPFLGMKSIFGFLGISYLTFKAVQTVMEIRDGVIKDF NPWFFLNFLAFFPTISSGPIDRYRRYKKDYYSVPNKEKYIQLLEKGLHYIFLGFLYDFMLSYFFGTVLLP GIKREVIASTGVSLALVEYMYVYSMYLFFNFAGYSLFAVGTSYFMGIETPMNFNQPFKSKNIKEFWNRWH MTLSFWFRDYVYMRLVFFFMKKKVFKNPKTTANITYILNMLLMGFWHGETWYYILYGFIHGVALVVNDWW LGYKKKHKDVVPHNKFTELFAIFITFNFVCFTFLIFSGILDFVKFR |

### Bactofensin LS1 is active at micromolar concentrations.

The availability of the structural gene and deduced peptide sequences facilitated the production of synthetic Bactofensin LS1. MS analysis revealed a mass of 2,784 Da for the synthetic form of the peptide, which displayed anti*-Listeria* and anti-S. *aureus* activity that was similar to that of the purified natural Bactofensin LS1 peptide. This suggests that the disulfide bond of the natural form of Bactofensin LS1 is not crucial for activity. As a consequence of this, and the fact that the synthetic approach provided access to larger quantities of peptide, the synthetic Bactofensin LS1 peptide was employed for further antimicrobial activity assays, which on this occasion took the form of more sensitive broth-based minimum inhibitory concentration 50 (MIC₅₀) assays. These investigations revealed that although concentrations of up to 50 µM of synthetic Bactofensin LS1 did not inhibit *E. coli* and *E. sakazakii* strains, a concentration of 5 µM was sufficient to inhibit the growth of both S. *aureus* DPC5246 and *L. monocytogenes* NCTC 11994 by 50% (Figure 3). This activity is comparable to that of the two-component lantibiotic lacticin 3147, which displayed a MIC₅₀ of 7-8 µM for the bovine mastitis isolate S. *aureus* DPC5245. However, the fact that Bactofensin LS1 is an unmodified antimicrobial peptide and thus can be readily generated in large quantities in a synthetic form may make this novel antimicrobial peptide a more favourable alternative to antibiotics for animal husbandry related applications. Bactofensin LS1 may also find applications in reducing the incidence of sensitive methicillin-resistant S. *aureus* in pigs which can act as carriers of this pathogen and which have been linked with its transmission to humans.

### The Bactofensin LS1 locus is a novel hyper-variable gene cluster characteristic of L. salivarius of porcine origin.

The ability of genetically distinct strains, and in several cases distinct species, to produce similar or identical antimicrobial peptides is a common phenomenon of LAB. It is evident from this study and others that there is a relatively high content of hyper-variable gene clusters in *L. salivarius.* It is now also apparent that DPC6502 contains many features not previously associated with *L. salivarius,* despite the current availability of two complete *L. salivarius* genome sequences and two draft genome assemblies (accession no. ACGT00000000 and AEBA00000000), all of which correspond to strains of human origin. To investigate if the Bactofensin LS1 locus described herein is among the hyper-variable loci of the flexible gene pool of *L*. *salivarius,* six further *L. salivarius* isolates of human and porcine intestinal origin were investigated for the presence of the Bactofensin LS1 structural gene. A PCR product corresponding to this gene could not be generated from the genomic DNA of the two strains of human origin, DPC6488 and DPC6196. However, all four porcine strains, (L. *salivarius* DPC6005, DPC6027, DPC6189, and 7.3) were positive for *bfls*1, and sequencing of the PCR products generated confirmed 100% identity with the Bactofensin LS1 structural gene of DPC6502 in each case. These strains also produce a two-component class IIb antimicrobial peptide, salivaricin P.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. An isolated antimicrobial peptide of SEQ ID NO: 2, or a variant thereof having at least 90% sequence identity with SEQ ID NO: 2, wherein the antimicrobial peptide of SEQ ID NO: 2, or the variant thereof, has potent antibacterial activity against *Listeria monocytogenes* and *Staphylococcus aureus.*

2. An isolated antimicrobial peptide, or variant thereof, of Claim 1 **characterised in that** it has antimicrobial activity against *Listeria monocytogenes* and *Staphylococcus aureus* of at least 10µM.

3. An isolated *Lactobacillus salivarius* DPC6502 strain as deposited with the National Collection of Industrial and Marine Bacteria under the Accession No. NCIMB 41840 on 9 May 2011, and variants thereof, wherein the variants retain the phenotypic characteristics of the isolated bacteria, and wherein the isolated bacteria and variants thereof express a antimicrobial peptide having potent antibacterial activity against *Listeria monocytogenes* and *Staphylococcus aureus.*

4. An isolated nucleic acid sequence encoding the isolated antimicrobial peptide, or variant thereof, of Claim 1 or Claim 2.

5. An isolated nucleic acid sequence as claimed in Claim 4 and having a sequence of SEQ ID NO: 1, or a variant thereof having at least 90% sequence identity with SEQ ID NO: 1, and wherein the nucleic acid of SEQ ID NO: 1, or the variant thereof, encode a antimicrobial peptide having potent antibacterial activity against *Listeria monocytogenes* and *Staphylococcus aureus.*

6. An isolated immunity protein having an amino acid sequence of SEQ ID NO: 8, or an isolated variant thereof having at least 90% sequence identity with SEQ ID NO: 8, wherein the isolated protein or variant thereof is capable of conferring immunity on a host strain to the antibacterial effects of the antimicrobial peptide of the invention.

7. A nucleic acid sequence encoding the isolated cognate immunity protein, or variant thereof, of Claim 6.

8. A recombinant vector comprising one or more of: a nucleic acid sequence, or variant thereof, of Claim 4 or Claim 5; a nucleic acid sequence encoding an antimicrobial peptide, or variant thereof, of Claim 1 or 2; and a nucleic acid sequence encoding an immunity protein, or variant thereof, of Claim 6.

9. A recombinant vector of Claim 8 comprising a nucleic acid sequence of Claim 4 or Claim 5 and a nucleic acid sequence of Claim 7.

10. A host cell transformed by a recombinant vector of the Claim 8 or 9.

11. An antimicrobial peptide, or variant thereof, of Claim 1 or Claim 2, for use as a medicament.

12. An antimicrobial peptide, or variant thereof, of Claim 1 or Claim 2, for use as an antibacterial agent or an antibiotic.

13. An antimicrobial peptide, or variant thereof, of Claim 1 or Claim 2, for use in reducing the incidence of sensitive methicillin-resistant S. *aureus* in animals, especially pigs.

14. A pharmaceutical composition comprising an antimicrobial peptide, or variant thereof, of Claim 1 or Claim 2, in combination with a suitable pharmaceutical excipient.

15. A probiotic composition comprising the strain, or variant thereof, of Claim 3, or an antimicrobial peptide, or variant thereof, of Claim 1 or Claim 2.
